# FASCICULE DE BREVET EUROPEEN

(11) **EP 3 905 989 B1**
(45) Date de publication et mention de la délivrance du brevet: **28.01.2026**
(21) Numéro de dépôt: 20700039.9
(22) Date de dépôt: 03.01.2020
(51) Int. Cl.: A61F 2/06, A61F 2/07

(54) **PROCÉDÉ DE FABRICATION AUTOMATISÉE D'ENDOPROTHÈSE VASCULAIRE**
VERFAHREN ZUR AUTOMATISIERTEN HERSTELLUNG EINER GEFÄSSENDOPROTHESE
METHOD FOR AUTOMATED PRODUCTION OF A VASCULAR ENDOPROSTHESIS

(30) Priorité: 03.01.2019 FR 1900042
(43) Date de publication de la demande: 10.11.2021
(73) Titulaire: Fondation Hôpital Saint Joseph, 75014 Paris (FR)
(72) Inventeur: FABRE, Dominique, 92380 Garches (FR); HAULON, Stéphan, 75007 Paris (FR)
(74) Mandataire: Plasseraud IP
(86) Numéro de dépôt international: PCT/EP2020/050071
(87) Numéro de publication internationale: WO 2020/141211

(56) Documents cités:
- WO-A1-2017/158288
- WO-A2-2004/026183

## Description

### DOMAINE TECHNIQUE DE L'INVENTION

L'invention se rapporte aux dispositifs médicaux implantables, plus précisément aux endoprothèses médicales sur mesure utilisées en cardiologie ou en chirurgie vasculaire.

Elle concerne notamment un procédé de fabrication automatisée d'une endoprothèse sur mesure fenêtrée et/ou branchée, adaptée à une cavité naturelle pouvant comporter des bifurcations telle qu'une aorte.

### ETAT DE LA TECHNIQUE

Pour la fabrication d'endoprothèses, c'est-à-dire de prothèses destinées à une implantation à l'intérieur de l'organisme, on utilise le plus souvent un maillage prédécoupé fait en un métal biocompatible, éventuellement cousu sur une membrane de polymère.

La prothèse finale est destinée à s'étendre après implantation dans une cavité naturelle d'un patient. Une cavité naturelle d'un individu est par exemple un vaisseau sanguin. Le matériau synthétique de la prothèse forme une paroi qui présente une étanchéité aux fluides biologiques ainsi qu'une certaine rigidité après son expansion, et forme un passage pour des fluides naturels tels que du sang.

On peut par exemple traiter un anévrisme à l'aide d'une endoprothèse de forme tubulaire. La paroi de la prothèse doit présenter une certaine rigidité et des zones d'étanchéité afin de limiter les risques de fuites après la pose.

On peut également traiter d'autres lésions artérielles avec ce type de prothèses.

Les opérations de prise de mesures, puis de fabrication manuelle rallongent le délai de conception et de fabrication d'endoprothèses vasculaires destinées à des pathologies complexes jusqu'à de nombreuses semaines voire plusieurs mois.

Les branches de l'aorte constituent des bifurcations, qui nécessitent de concevoir une endoprothèse présentant des « fenêtres » ou des « branches » situées au niveau des intersections entre la voie principale et les branches d'aorte. A défaut, la circulation du sang est bloquée dans les branches d'aorte, une fois l'endoprothèse implantée chez le patient. La réalisation d'une telle prothèse rallonge encore les délais de conception et de fabrication, les fenêtres et/ou branches étant principalement réalisées par des découpes et des coutures manuelles sur une prothèse existante.

Pour bien connaître la position et l'orientation des branches d'aorte, une analyse d'images vasculaires, obtenues par exemple par scanner, par Imagerie par Résonance Magnétique ou par angiographie, est nécessaire.

Outre son temps de réalisation, la couture manuelle d'une paroi synthétique d'endoprothèse présente un inconvénient additionnel : des sutures manuelles sont réalisées, autour et à proximité des fenêtres ou des branches. Des zones de moindre étanchéité et de tenue mécanique plus faible sont créées au niveau de ces sutures.

Il existe donc un risque de fuites de sang hors de la paroi de la prothèse, à des endroits non prévus. La performance des endoprothèses actuelles peut donc être améliorée.

De plus, les particularités anatomiques des parois de la cavité naturelle à traiter, comme une angulation inhabituelle ou la présence de calcifications, ne peuvent pas être prises en compte de façon optimale lorsque la prothèse est fabriquée manuellement.

Pour accélérer le processus de conception et de fabrication des endoprothèses, pour réduire les risques de défaillance dues aux sutures mécaniques, pour mieux s'adapter aux anatomies difficiles, et pour améliorer la qualité de ces prothèses, il a été proposé de fabriquer un mandrin formant un moule à l'image de la cavité naturelle du patient. L'endoprothèse, éventuellement fenêtrée ou branchée, est fabriquée à partir de ce mandrin. La demande de brevet US 2013/0296998 décrit un tel procédé de fabrication, au cours duquel des perçages sont réalisés pour obtenir les fenêtres et les branches de la prothèse finale.

Les fenêtres et les branches sont positionnées sur le mandrin afin de correspondre au mieux à l'anatomie du patient. Elles peuvent être complétées après démoulage du mandrin sur un autre mandrin correspondant à chaque branche.

La demande internationale WO 2017/158288 A1 décrit un procédé de fabrication d'une endoprothèse tubulaire pour implantation dans un conduit anatomique, qui comprend des étapes de génération d'un modèle virtuel tridimensionnel dudit conduit anatomique à partir de données dimensionnelles acquises par imagerie médicale; réduction des dimensions de ce modèle virtuel par enlèvement d'une couche d'épaisseur e à partir de toute la surface périphérique dudit modèle virtuel; réalisation d'une préforme selon ledit modèle virtuel tridimensionnel; formation, sur la surface périphérique de la préforme, d'une enveloppe externe tubulaire en un matériau élastomère biocompatible, d'épaisseur e, par trempage de la préforme dans une solution d'un précurseur réticulable dudit matériau élastomère, et réticulation; et démoulage de l'enveloppe externe tubulaire ainsi formée.

### PRESENTATION GENERALE DE L'INVENTION

Il existe ainsi un besoin pour un procédé de fabrication d'endoprothèses fenêtrées sur mesure correspondant à l'anatomie d'une cavité naturelle, qui soit automatisé, rapide et plus adapté à l'anatomie précise de la lésion traitée. Le coût peut également être optimisé par rapport à une prothèse avec de nombreuses sutures manuelles.

Le procédé doit faciliter la fabrication d'endoprothèses en série à partir d'images tridimensionnelles de patients et l'obtention de prothèses de haute qualité et ayant des propriétés mécaniques améliorées.

Un besoin additionnel existe pour un procédé de fabrication d'une endoprothèse fenêtrée ou branchée de type couvert comportant une paroi synthétique, avec ou sans maillage métallique. Les renforcements au niveau des fenêtres et/ou le maillage métallique doivent présenter une bonne cohésion avec la paroi prothétique dans les zones de superposition avec cette paroi.

A ce titre, l'invention concerne selon un premier aspect un procédé de fabrication d'une endoprothèse vasculaire destinée à être insérée dans une cavité naturelle d'un individu, à partir d'un modèle tridimensionnel de la cavité naturelle, ledit modèle comprenant une voie principale et au moins une branche s'étendant depuis la voie principale, le procédé comprenant des étapes de :
obtention d'une structure d'empreinte conformée pour suivre une forme de la voie principale,
la structure d'empreinte comprenant au moins un emplacement en correspondance avec l'intersection du modèle tridimensionnel,
fabrication d'une paroi de prothèse au moyen de la structure d'empreinte, la paroi de prothèse étant en polymère, une fenêtre de prothèse ou une branche de prothèse étant fabriquée au niveau de l'emplacement,
obtention de l'endoprothèse vasculaire comprenant la fenêtre de prothèse ou la branche de prothèse.

Selon ce procédé, la fabrication d'une prothèse couverte (comprenant une paroi en polymère) comprend l'obtention d'une structure d'empreinte qui prend en considération le positionnement anatomique des différentes branches aortiques.

A partir de cette structure d'empreinte, dans laquelle un emplacement de branche ou de fenêtre repère une intersection entre une voie principale et une branche de voie, on obtient une endoprothèse fenêtrée ou branchée comprenant une fenêtre de prothèse ou une branche de prothèse.

La prothèse ainsi obtenue peut ensuite être implantée chez un patient et on peut en contrôler le positionnement au sein de la cavité naturelle du patient lors de l'implantation (typiquement à l'aide d'un système de largage). La position initiale d'implantation peut être choisie de sorte que des fenêtres ou branches de la prothèse soient, après expansion de la prothèse, superposés aux intersections entre la voie principale et les branches vasculaires.

Un avantage du procédé est d'obtenir de manière automatisée et rapide une endoprothèse vasculaire sur mesure.

Les coûts et les délais de fabrication sont très limités, notamment car l'acquisition du modèle de prothèse et la fabrication de la prothèse nécessitent peu d'intervention humaine.

Le temps de conception et de fabrication de l'endoprothèse fenêtrée peut ainsi être réduit à partir de l'obtention des images médicales.

Dans le cas où l'endoprothèse vasculaire comprend en outre un maillage métallique et/ou un ou plusieurs renforcements au niveau des intersections, on peut également solidariser le maillage métallique et/ou la ou les renforcement(s) avec la paroi en polymère, sans couture lors de la fabrication de la paroi.

Le procédé de fabrication de l'invention peut comprendre en outre les caractéristiques additionnelles et non limitatives suivantes, prises seules ou dans l'une quelconque des combinaisons techniquement possibles :
- le procédé comprend une étape de placement d'un renforcement sur l'emplacement de la structure d'empreinte, préalablement à la fabrication de la paroi de prothèse, la fenêtre de prothèse ou la branche de prothèse étant fabriquée sur le renforcement.

Dans cette dernière variante, un premier avantage est d'éviter de réaliser des sutures manuelles pour solidariser les renforcements de la prothèse avec la paroi en polymère.

On évite ainsi de créer des zones de faiblesse mécanique dans l'endoprothèse finale après expansion, propices à d'éventuelles ruptures ou fuites de fluide.

Les renforcements de la prothèse sont placés avec une précision de préférence au moins millimétrique au niveau des intersections entre la voie principale vasculaire et les branches vasculaires.

Un deuxième avantage de cette variante est d'améliorer encore davantage la concordance anatomique entre les fenêtres ou branches de la prothèse obtenue à l'issue du procédé et les intersections entre la voie principale réelle et les branches réelles de la cavité naturelle ;
- la structure d'empreinte est obtenue par impression tridimensionnelle, par exemple par dépôt de matière fondue ou FDM ;
- la structure d'empreinte est pleine et la paroi de prothèse est fabriquée par moulage par-dessus la structure d'empreinte ;
- le procédé comprend une étape intermédiaire de placement, le long de la structure d'empreinte, d'un maillage métallique destiné à s'étendre le long d'au moins une partie de l'endoprothèse vasculaire, la paroi de prothèse étant réalisée autour du maillage métallique ;
- ledit emplacement de la structure d'empreinte comprend un volume cylindrique formant une protrusion, le placement du renforcement comprenant le positionnement du renforcement contre la protrusion.

Un avantage technique associé est de faciliter et de rendre plus précis le positionnement du renforcement, par exemple de l'ergot, en correspondance avec l'intersection du modèle tridimensionnel ;
- la fabrication de la paroi de prothèse comprend un enroulement de filaments de polymère autour de la structure d'empreinte et/ou un enrobage de la structure d'empreinte par des couches de polymère ;
- la structure d'empreinte est entraînée en rotation pendant la fabrication de la paroi de prothèse ;
- le renforcement comprend un ergot, de préférence fabriqué au moins en partie en nitinol ;
- des capteurs sont placés contre la structure d'empreinte avant la fabrication de la paroi de prothèse ;
- la paroi de prothèse est composée en majorité de polytétrafluoro-éthylène dit PTFE ou de poly-téréphtalate d'éthylène dit PETE ;
- le renforcement est composé en majorité de nitinol ;
- le procédé comprend une étape supplémentaire de fabrication d'une paroi de branche au moyen d'une empreinte de branche, la paroi de branche étant en polymère, l'empreinte de branche étant placée au niveau de l'emplacement lors de la fabrication de la paroi de branche, l'endoprothèse vasculaire obtenue comprenant la paroi de branche reliée à la paroi de prothèse ;
- le modèle tridimensionnel de la cavité naturelle comprend une discontinuité de la voie principale, telle qu'une tortuosité, une calcification ou un thrombus.

Selon un deuxième aspect, l'invention se rapporte à une endoprothèse vasculaire, par exemple une endoprothèse d'aorte, comprenant une fenêtre et/ou une branche, l'endoprothèse étant obtenue par un procédé de fabrication tel que défini ci-avant.

Selon un troisième aspect, l'invention concerne un ensemble de fabrication d'une endoprothèse vasculaire, l'ensemble étant configuré pour mettre en œuvre un procédé de fabrication tel que défini ci-avant, l'ensemble comprenant :
une unité d'obtention d'une structure d'empreinte par impression tridimensionnelle ;
une unité de fabrication de paroi de prothèse à partir de la structure d'empreinte ;
une unité de traitement, configurée pour contrôler la fabrication de la structure d'empreinte par l'unité de fabrication en fonction d'un modèle tridimensionnel de cavité naturelle, l'unité de traitement comprenant une mémoire pour sauvegarder le modèle tridimensionnel.

L'ensemble de fabrication d'endoprothèse selon l'invention peut fonctionner de manière automatisée avec une intervention très minime du praticien. En effet, le modèle tridimensionnel est utilisé pour obtenir une structure d'empreinte, puis pour fabriquer une endoprothèse fenêtrée ou branchée adaptée à la morphologie de la cavité.

De plus, un tel ensemble atteint une rapidité supérieure à celle des systèmes de l'art antérieur puisque la fabrication de la paroi de prothèse est réalisable directement sur la structure d'empreinte, sans qu'il ne soit nécessaire de réaliser un perçage ou une couture manuelle.

De façon optionnelle et non limitative, l'ensemble de fabrication d'endoprothèse comprend en outre une unité d'acquisition de modèle configurée pour acquérir des images d'une cavité naturelle d'un patient et pour générer à partir desdites images un modèle tridimensionnel de la cavité naturelle.

Cet ensemble de fabrication d'endoprothèse comprend de manière optionnelle et non limitative une unité d'acquisition de modèle configurée pour acquérir des images d'une cavité naturelle d'un patient et générer à partir desdites images un modèle tridimensionnel de la cavité naturelle.

### PRESENTATION GENERALE DES FIGURES

D'autres caractéristiques, buts et avantages de l'invention ressortiront de la description qui suit, qui est purement illustrative et non limitative, accompagnée des dessins annexés parmi lesquels :
La **Figure 1** est une représentation schématique d'un ensemble de fabrication d'une endoprothèse vasculaire selon un exemple de réalisation de l'invention ;
La **Figure 2** représente les étapes d'un procédé de fabrication d'une endoprothèse selon un mode de réalisation ;
La **Figure 3** est une vue schématique d'un modèle tridimensionnel d'aorte généré par ordinateur, comprenant des annotations ;
La **Figure 4** est une vue d'une structure d'empreinte selon une première variante, fabriquée conformément au modèle de la Figure 3 ;
La **Figure 5** est une vue d'une structure d'empreinte selon une deuxième variante.
La **Figure 6** représente la structure d'empreinte de la Figure 4 après le placement de renforcements et d'un maillage métallique ;
La **Figure 7** est une vue en perspective d'une prothèse couverte fenêtrée avant expansion, obtenue à l'issue du procédé de fabrication ;
La **Figure 8** représente l'endoprothèse de la Figure 7, après expansion dans l'aorte ;
La **Figure 9** est une vue d'une structure d'empreinte selon une troisième variante.

### DESCRIPTION DETAILLEE DE MODES DE REALISATION

On décrit ci-après un procédé de fabrication d'une endoprothèse comprenant une paroi extérieure destinée à s'étendre le long d'une voie principale d'une cavité naturelle ainsi que des perforations au niveau de branches de la cavité naturelle s'étendant depuis la voie principale.

La cavité naturelle est une cavité d'un patient humain ou animal.

Une telle prothèse constitue un dispositif médical implantable (ou DMI) expansible, pouvant adopter une position finale au sein d'une cavité naturelle qui est différente de sa position initiale (avant déploiement), et qui est également différente de sa position au repos.

La paroi extérieure et les perforations de la prothèse sont conçues pour ne pas obstruer l'intersection entre la voie principale et les branches, après expansion de la prothèse.

On comprendra aisément que les avantages de l'invention, relatifs à l'utilisation d'une structure d'empreinte reproduisant la lumière vasculaire, s'appliquent de la même manière pour une prothèse comprenant une ou plusieurs fenêtres (prothèse fenêtrée) et/ou comprenant une ou plusieurs branches (prothèse branchée). Par « branche de prothèse » on entend une paroi s'étendant le long d'une branche de la cavité naturelle, ladite paroi étant solidaire de la paroi de prothèse s'étendant le long de la voie principale de la cavité naturelle.

On peut également concevoir, à l'aide de la structure d'empreinte de l'invention, une prothèse destinée à une cavité naturelle qui ne comprendrait pas de branche.

Le mot « prothèse » se rapporte à une endoprothèse vasculaire (précisément aortique) dans ce qui suit. Par ailleurs, dans toute la suite, des éléments similaires seront désignés avec les mêmes références numériques dans la description et sur les figures annexées.

### Ensemble de fabrication d'une endoprothèse vasculaire

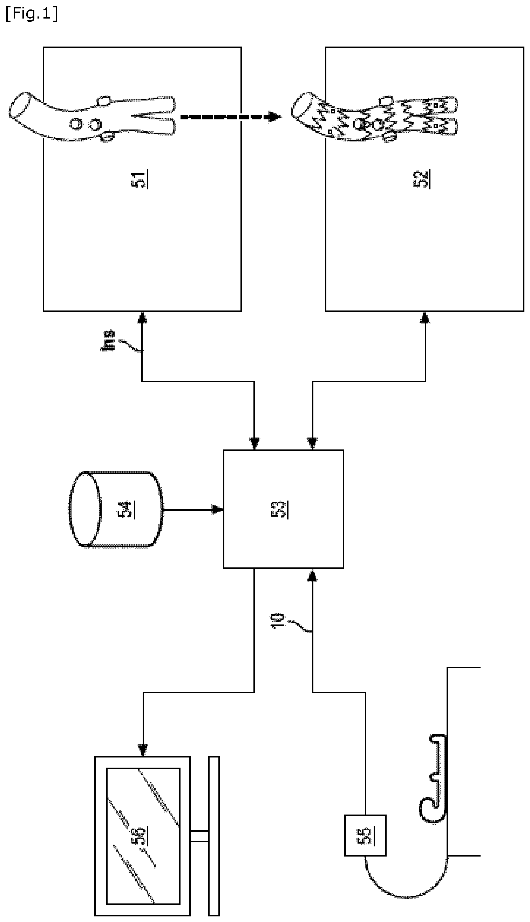

On a représenté en **Figure 1** un ensemble de fabrication d'une prothèse selon un mode de réalisation de l'invention.

Ledit ensemble peut être installé dans un site de fabrication en série de prothèses, dans un hôpital, dans un laboratoire médical, une entreprise, etc. Il comprend une unité 51 d'obtention d'une structure d'empreinte et une unité 53 de moulage et de démoulage de membrane de prothèse, commandées par une unité 52 de traitement.

L'unité 51 est configurée pour réaliser sur commande, et à partir d'un modèle tridimensionnel généré par ordinateur, une structure d'empreinte. Par « structure d'empreinte » on entend un support, plein ou creux, contre lequel une membrane et/ou un maillage métallique peuvent être fabriqués, par exemple moulés, pour prendre la forme souhaitée de la prothèse avant expansion. La structure d'empreinte constitue ainsi une reproduction de la lumière vasculaire de la cavité naturelle qu'il s'agit de traiter.

De façon très avantageuse, l'unité 51 est configurée pour fabriquer des structures d'empreinte par impression tridimensionnelle.

Des avantages de l'impression tridimensionnelle sont sa rapidité d'exécution, son caractère répandu dans le domaine des matériaux et sa précision.

En alternative, l'unité 51 d'obtention de structures d'empreinte peut être configurée pour adapter des structures d'empreintes déjà existantes à des cavités naturelles de patients, par exemple par découpe. On peut ainsi envisager de réutiliser plusieurs fois une seule structure d'empreinte. L'objectif reste néanmoins de faire de la prothèse sur mesure. La prothèse obtenue par le procédé de fabrication décrit ci-après est donc de préférence à usage unique.

L'unité 52 est configurée pour réaliser sur commande la fabrication (typiquement le moulage, puis le démoulage) d'une prothèse synthétique à partir d'une structure d'empreinte, selon les modalités qui seront décrites ci-après.

L'unité de traitement 53 est configurée pour commander l'unité 51 et l'unité 52. A ce titre, l'unité 53 comprend de préférence un processeur sur lequel sont programmées des instructions de code pour transférer des instructions aux unités 51 et 52.

Notamment, l'unité 51 est configurée pour recevoir de l'unité de traitement 52 des instructions pour réaliser l'impression tridimensionnelle de structures d'empreinte.

En alternative, deux unités de traitement séparées peuvent contrôler les opérations des unités 51 et 52.

Pour la communication d'instructions et de données informatiques, une liaison électronique existe entre l'unité de traitement 53 et chacune des unités 51 et 52, selon tout type de liaison avec ou sans fil.

De préférence, l'unité de traitement 53 est en outre configurée pour communiquer avec une unité 55 d'acquisition de modèle tridimensionnel des cavités naturelles d'un patient.

L'unité 55 est configurée pour acquérir des vues permettant de reconstituer une image tridimensionnelle d'une région d'intérêt, par imagerie à rayons X, par angiographie ou par toute autre technique d'imagerie médicale adaptée à la cavité naturelle à traiter.

De façon alternative, les vues permettant de reconstituer le modèle tridimensionnel peuvent être issues d'une base de données distante, ici notée 54.

On peut prévoir en outre un dispositif d'affichage 56 connecté à l'unité de traitement 53 et/ou à d'autres éléments de l'ensemble de la Figure 1, comprenant une interface graphique.

### Procédé de fabrication d'une endoprothèse vasculaire

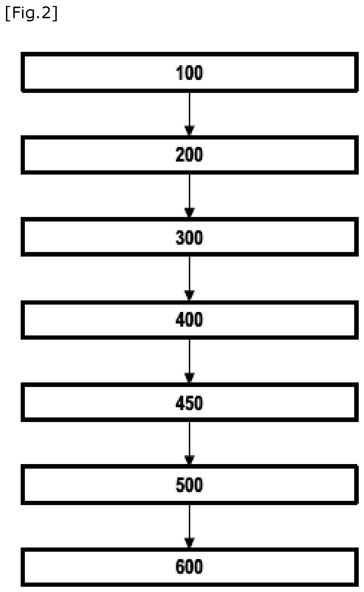

La **Figure 2** représente les étapes d'un procédé de fabrication de prothèse fenêtrée selon un mode de réalisation de l'invention.

L'ensemble décrit ci-avant en relation à la Figure 1 est apte à mettre en œuvre ce procédé.

A une étape 100 optionnelle, des images médicales d'une cavité naturelle à traiter sont acquises et enregistrées par l'unité 55 d'acquisition. Les images sont par exemple acquises par angiographie ou par rayons X.

Dans toute la suite, la cavité naturelle à traiter sera une aorte d'un individu. On se situe dans l'ensemble de l'aorte, depuis l'aorte ascendante et la crosse au niveau de ses différentes branches, mais également dans les portions suivantes de l'aorte au niveau des branches thoraco-abdominales. Ces branches aortiques peuvent également être réalisées pour d'autres conformations anatomiques particulières : montages prothétiques chirurgicaux, dissections aortiques, etc.

En alternative, on peut exploiter, pour la suite du procédé de fabrication de prothèse, des images médicales déjà disponibles, par exemple enregistrées dans une base de données distante.

Les images peuvent être bidimensionnelles ou tridimensionnelles.

A une étape 200, un modèle tridimensionnel d'une cavité naturelle d'un patient est généré à partir de vues de la cavité naturelle à traiter. Le modèle tridimensionnel est par exemple constitué de sommets tridimensionnels placés dans un repère virtuel (X, Y, Z). Le modèle peut comprendre en outre des éléments de surface joignant lesdits sommets. La surface formée par les éléments de surface correspond ainsi à la surface des parois de la cavité naturelle.

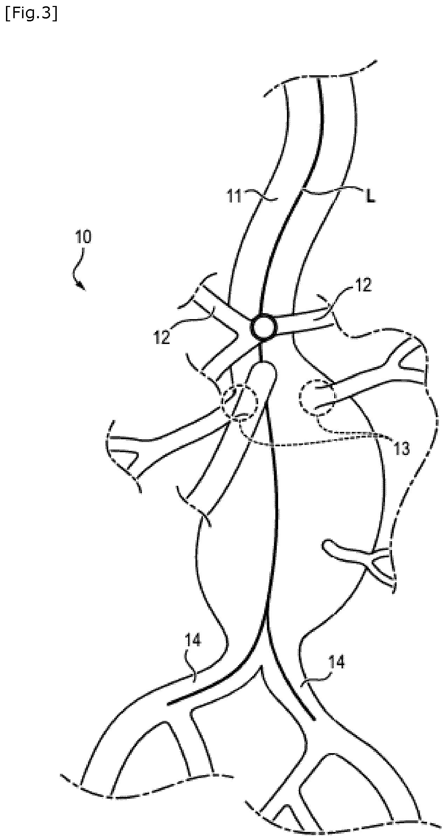

On a représenté un exemple de modèle tridimensionnel 10 sur la Figure 3. L'aorte comprend une voie principale 11 et une pluralité de branches de voie s'étendant depuis la voie principale. Deux desdites branches ont la référence 12 sur la figure. L'aorte comprend en outre deux artères iliaques 14 qui prolongent la voie principale 11 par le bas.

On constate ici que le modèle tridimensionnel 10 a été obtenu à partir de vues d'une aorte d'un individu qui présente un anévrisme. L'anévrisme s'étend par exemple des artères iliaques 14 jusqu'à la zone de l'aorte abdominale localisée au-dessus des branches 12.

On a également représenté sur le modèle de la Figure 2 des zones 13 qui correspondent à des intersections entre la voie principale 11 de l'aorte abdominale et les branches aortiques 12. Pour ne pas bloquer le passage du sang dans les branches aortiques 12, les fenêtres de la prothèse doivent être alignées avec les intersections 13 après expansion de la prothèse.

Pour traiter l'anévrisme, on souhaite fabriquer une prothèse fenêtrée dont une la paroi synthétique créera, après implantation et expansion de la prothèse, un passage de sang dont une section est plus réduite que la section de l'anévrisme.

De manière avantageuse, l'unité d'acquisition du modèle tridimensionnel peut en outre être configurée pour détecter une ligne centrale L du modèle tridimensionnel de la cavité naturelle, le long de laquelle la prothèse fenêtrée est destinée à s'étendre après implantation.

En alternative, au lieu de générer un modèle de la cavité naturelle lors de la fabrication de la prothèse, on peut utiliser un modèle acquis antérieurement, ou encore utiliser un modèle générique.

A une étape 300, une structure d'empreinte est obtenue à partir du modèle tridimensionnel de la cavité naturelle. La structure d'empreinte permet la fabrication d'une paroi de prothèse. De manière avantageuse, la structure d'empreinte est un mandrin de moulage.

La structure d'empreinte est conformée à la lumière vasculaire, ici à la lumière aortique. On entend par « conformée » qu'une paroi de la structure d'empreinte, correspondant à la zone sur laquelle sera moulée une paroi de la prothèse par la suite, suit une forme de la paroi interne de la lumière aortique.

De préférence, on fabrique à partir de zéro une nouvelle structure d'empreinte après avoir obtenu le modèle tridimensionnel.

Dans un mode de réalisation, la structure d'empreinte est pleine. Un volume de la structure d'empreinte correspond à un volume interne de la lumière vasculaire. La structure d'empreinte a ainsi une forme générale constituée d'un ou plusieurs tubes.

Dans l'exemple qui va suivre, la structure est un mandrin 20 plein.

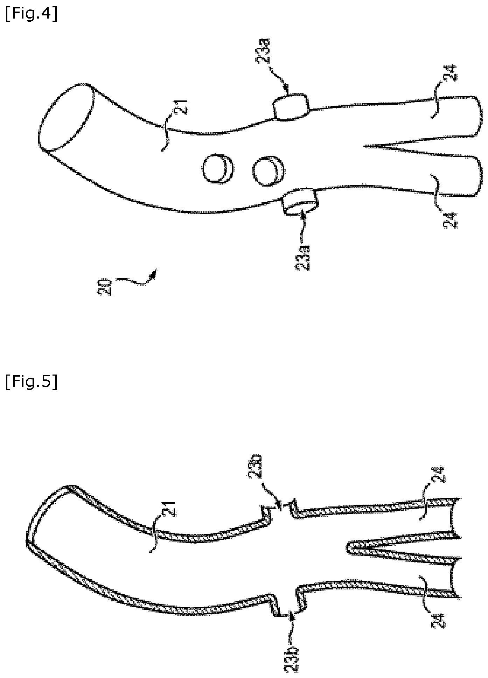

On a représenté en **Figure 4** un mandrin 20 conforme au modèle tridimensionnel de la Figure 3.

Ce mandrin est constitué d'un premier volume 21 tubulaire correspondant à l'aorte abdominale, et de deux volumes 24 tubulaires correspondant aux artères iliaques.

Les particularités anatomiques de la cavité naturelle à traiter (par exemple calcifications, tortuosités, thrombus, anévrismes) sont prises en compte pour l'obtention du mandrin.

Le mandrin comprend des emplacements spécifiques qui correspondent aux intersections 13 du modèle tridimensionnel entre la voie principale et les branches.

Chacun desdits emplacements permet, au cours du moulage ultérieur de la paroi de prothèse à partir du mandrin 20, de former une fenêtre ou une branche de prothèse sur la zone prévue pour le passage des vaisseaux de la cavité naturelle après expansion de la prothèse.

Ici, les emplacements correspondant aux branches 12 du modèle sont des protrusions 23a. Les protrusions 23a sont des volumes cylindriques de petite longueur, s'étendant depuis la paroi du volume 21 du mandrin.

En alternative, les emplacements correspondant aux intersections 13 sont des perforations.

De façon très avantageuse, la structure d'empreinte (ici le mandrin) est obtenue par impression tridimensionnelle.

Outre sa rapidité, l'impression tridimensionnelle présente l'avantage de permettre de se conformer très précisément au modèle tridimensionnel de cavité naturelle précédemment obtenu. De plus, l'impression tridimensionnelle peut être très facilement automatisée.

Le mandrin 20 est par exemple réalisé en polymère ou en métal.

Pour faciliter le démoulage ultérieur du mandrin, le mandrin peut être constitué de plusieurs volumes qui peuvent facilement être séparés par les extrémités. Par exemple, ces volumes sont collés ou attachés entre eux pendant le moulage, et peuvent ensuite être séparés.

Selon un mode alternatif, la structure d'empreinte de moulage peut ne pas être pleine, mais creuse. La structure d'empreinte est alors un moule creux. La paroi de prothèse sera dans ce cas réalisée contre une paroi intérieure du moule.

On a représenté en **Figure 5** un moule selon ce mode alternatif. Le moule comprend une partie 21 de moule correspondant à l'aorte abdominale, ainsi que des parties 24 de moule dans le prolongement de la partie 21, les parties 24 correspondant aux artères iliaques.

Pour obtenir les emplacements destinés au positionnement des renforcements et au moulage des fenêtres, des bouches 23b peuvent être présentes dans la paroi interne du moule.

En variante, pour l'obtention de la structure d'empreinte conforme au modèle tridimensionnel, on utilise comme point de départ une structure d'empreinte déjà existante, par exemple un mandrin. Pour assurer que le mandrin corresponde à la cavité naturelle à traiter, on peut réaliser des déformations ou des découpes du mandrin.

De retour à la Figure 2, le procédé de fabrication de la prothèse fenêtrée comprend une étape essentielle 400 de positionnement d'au moins un renforcement au niveau d'un emplacement de la structure d'empreinte.

Comme indiqué ci-avant, les emplacements de la structure d'empreinte permettent de former des fenêtres ou branches de la prothèse, pour permettre le passage du sang dans les branches aortiques. De façon avantageuse, des renforcements sont positionnés contre une paroi de la structure d'empreinte pour garantir l'étanchéité des zones qui entourent les fenêtres ou les branches de la prothèse finale.

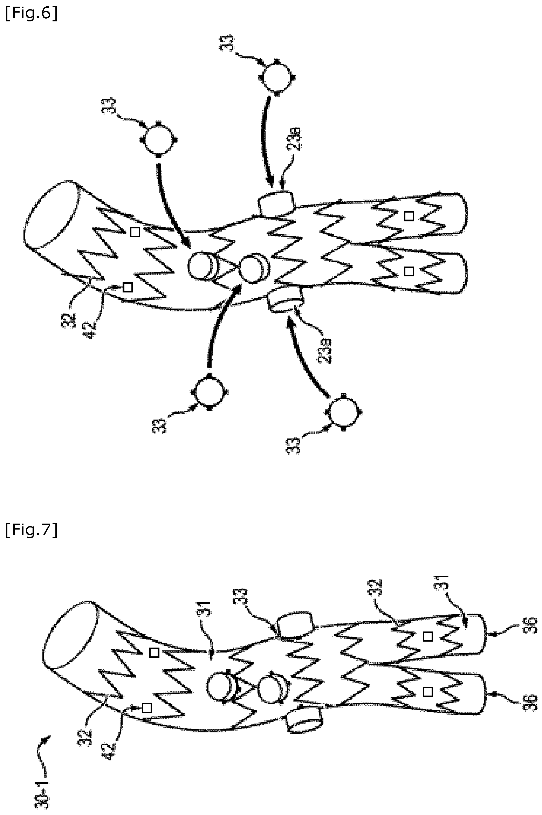

On a représenté en Figure 6 le mandrin 20 après positionnement de plusieurs renforcements à l'étape 400.

Les renforcements sont ici des ergots 33 placés contre les protrusions qui correspondent aux intersections entre la voie principale et les branches aortiques. Notamment, des ergots sont placés autour des deux protrusions 23a.

Les ergots sont ici des anneaux de faible épaisseur, de préférence une épaisseur inférieure à la longueur des protrusions. Un matériau des ergots est de préférence un métal biocompatible tel que du nitinol, ou bien un polymère.

De façon optionnelle et avantageuse, on place également à une étape 450 des fils 32 de maillage métallique expansible autour du mandrin. Ce maillage métallique est par exemple constitué de nitinol ou en alternative d'un autre alliage biocompatible.

Par exemple, on place autour du mandrin un stent métallique fabriqué préalablement, de forme tubulaire. On peut mettre bout à bout plusieurs stents métalliques si nécessaire (notamment dans l'exemple de la Figure 7, dans lequel l'extrémité basse du mandrin comprend deux jambes qui correspondent aux deux artères iliaques).

Ce maillage métallique 32 est destiné à s'étendre le long d'au moins une partie de l'endoprothèse vasculaire fenêtrée, comme il sera vu ci-après. Le maillage pourrait en alternative être placé sur le mandrin avant les renforcements 33.

Ce maillage métallique est avantageux pour obtenir une prothèse plus rigide et qui présente une meilleure tenue mécanique. Des zones d'étanchéité sont ainsi obtenues dans les zones où le calibre de l'aorte est normal comparativement à la zone pathologique.

De façon optionnelle, on place des capteurs 42 sur le mandrin. Ces capteurs sont destinés à être incorporés dans la paroi prothétique et à fonctionner au cours du cycle de vie de la prothèse.

De retour à la Figure 2, le procédé de fabrication de la prothèse fenêtrée se poursuit par la fabrication de la paroi synthétique de la prothèse à l'étape 500.

La fabrication est ici réalisée par moulage à partir de la structure d'empreinte, ici à partir du mandrin 20.

De façon avantageuse, la paroi prothétique est fabriquée en polymère. Le polymère choisi permet de préférence une expansion de la prothèse après son implantation dans une cavité naturelle.

La prothèse ainsi moulée constitue une paroi expansible de l'endoprothèse fenêtrée, formant un passage de sang. Ici, la cavité naturelle est affectée d'un anévrisme ; la prothèse réalisée comprend une paroi de section plus faible que la section de l'anévrisme.

Dans le cas où l'endoprothèse finale doit comporter un maillage métallique le long d'au moins une partie de sa longueur, la paroi prothétique est réalisée autour du maillage métallique de sorte à incorporer les fils de ce maillage.

Ici, le mandrin étant plein, la paroi prothétique est réalisée par-dessus le mandrin et autour des renforcements 32 et des fils 33 de maillage métallique.

Si la structure d'empreinte est creuse (comme sur la Figure 5), la paroi peut être moulée à l'intérieur de la structure d'empreinte par des techniques de moulage classiques, pour que la paroi soit conformée à la paroi intérieure du moule.

Plusieurs techniques peuvent être envisagées pour la fabrication de la paroi de prothèse à l'étape 500 :
- Enroulement ou enrobage du mandrin par des couches et/ou filaments de matériaux synthétiques (par exemple du polytétrafluoro-éthylène, dit PTFE) autour du mandrin, et coulage d'une matrice en polymère incorporant les filaments et incorporant également les renforcements et les fils de maillage métallique.

Au cours de l'enroulement et/ou de l'enrobage, la structure d'empreinte est, selon une variante possible du procédé de fabrication, entraînée en rotation, par exemple autour d'un axe longitudinal de la structure d'empreinte ;
- Déformation d'un tube en polymère préexistant pour se conformer aux parois du mandrin, en incorporant les renforcements de fenêtres et les fils de maillage métallique dans le tube en polymère ;
- Projection de polymères sur le mandrin en plusieurs couches en incorporant les fils de maillage métallique ou l'ensemble de la structure métallique de renforcement ;
- Utilisation d'une presse de contre-moulage pour venir appliquer les couches de polymère appliquées sur le mandrin ;
- Combinaison de ces différentes techniques.

Pour la mise en œuvre du moulage, le matériau polymère constitutif de la paroi 31 est porté à une température suffisamment élevée pour être déformable.

Un matériau polymère avantageux pour le moulage de la prothèse est le polytétrafluoro-éthylène, aussi dit PTFE. On peut aussi utiliser de polytéréphtalate d'éthylène, dit PETE.

Par la suite, la prothèse est démoulée à une étape 600.

Si la structure d'empreinte est un mandrin plein, le démoulage comprend le retrait du mandrin sans endommager la paroi de prothèse qui l'entoure.

Si la structure d'empreinte est un moule creux, il suffit de retirer le moule, une fois la prothèse rigidifiée.

On a représenté en **Figure 7** un exemple d'endoprothèse vasculaire fenêtrée obtenue après le moulage et le démoulage, à partir du mandrin de la Figure 6.

Ici, l'endoprothèse fenêtrée 30-1 est visible avant expansion. Il s'agit de l'état au repos de la prothèse, avant son implantation. Dans cet état, la prothèse n'est pas contrainte mécaniquement.

La prothèse comprend ainsi une paroi 31 en polymère. Les renforcements 33, les fils 32 de maillage et les capteurs 42 sont incorporés dans la paroi.

Ainsi, il n'est pas nécessaire que la prothèse comprenne des sutures pour coudre les parties métalliques.

De plus, la prothèse se termine vers le bas par deux passages 36 correspondant aux artères iliaques.

On notera que, de manière avantageuse, une extension radiale de la structure d'empreinte 20 ainsi que, par voie de conséquence, une extension radiale de la paroi 31 en polymère obtenue, sont toutes deux supérieures à une extension radiale de la voie principale 11 du modèle tridimensionnel 10 de la cavité naturelle à traiter.

Une « extension radiale » de la voie principale 11 du modèle tridimensionnel 10 est repérée par rapport à la ligne centrale L (illustrée sur la Figure 3) de la voie principale 11. Sur un plan de section donné transversal à la ligne centrale L, l'extension radiale de la voie principale 11 est égale à la distance maximale entre deux points de la voie principale 11 situés dans ledit plan de section.

Une « extension radiale » de la structure d'empreinte 20, ainsi qu'une « extension radiale » de la paroi 31 de la prothèse 30-1 obtenue, sont toutes deux repérées de manière similaire par rapport à une courbe longitudinale. La courbe longitudinale de la structure d'empreinte 20 est l'image de la ligne centrale L. La courbe longitudinale de la paroi 31 est également l'image de la ligne centrale L.

De préférence, l'extension radiale de la structure d'empreinte 20 est comprise entre 101% et 130% de l'extension radiale de la voie principale 11 de la cavité naturelle, de manière encore préférentielle entre 110% et 120% de l'extension radiale de la voie principale 11 de la cavité naturelle.

Autrement dit, la prothèse 30-1 obtenue est légèrement surdimensionnée comparativement au modèle tridimensionnel 10. On peut également parler d'un « oversizing » de la prothèse 30-1.

Un avantage associé est de promouvoir une meilleure apposition de la prothèse 30-1 obtenue contre les parois de la cavité naturelle, après implantation de la prothèse 30-1. On renforce de cette manière l'étanchéité de la prothèse obtenue vis-à-vis d'écoulements de fluides biologiques au sein de la cavité naturelle, tels que du sang.

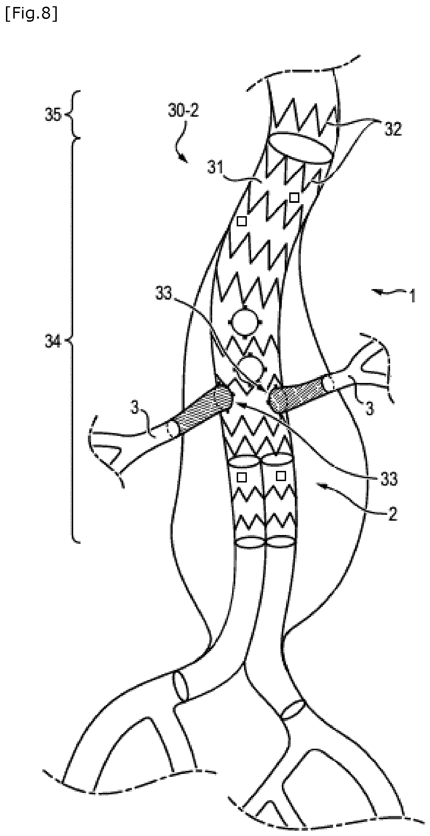

La **Figure 8** représente la même endoprothèse fenêtrée dans un état 30-2 après expansion dans l'aorte 1 d'un patient.

L'aorte 1 comprend la voie principale 2 et les branches 3.

Après son implantation via un dispositif d'implantation (par exemple un cathéter), la prothèse subit les contraintes mécaniques exercées par les parois environnantes de la cavité. Sous l'effet de ces contraintes, la prothèse peut être compressée, ce qui provoque une diminution localisée de sa section et un allongement localisé.

On a représenté une longueur 34 d'extension de la paroi 31 et une longueur 35 d'extension du maillage métallique au-delà des bords de la paroi 31, après expansion de la prothèse dans l'aorte. La paroi s'étend ici sur environ 90% de la longueur totale de la prothèse après expansion.

On a ainsi réalisé une endoprothèse vasculaire sur mesure, très fidèle à l'anatomie de la cavité naturelle à traiter et dont les propriétés mécaniques (étanchéité, tenue mécanique) sont améliorées.

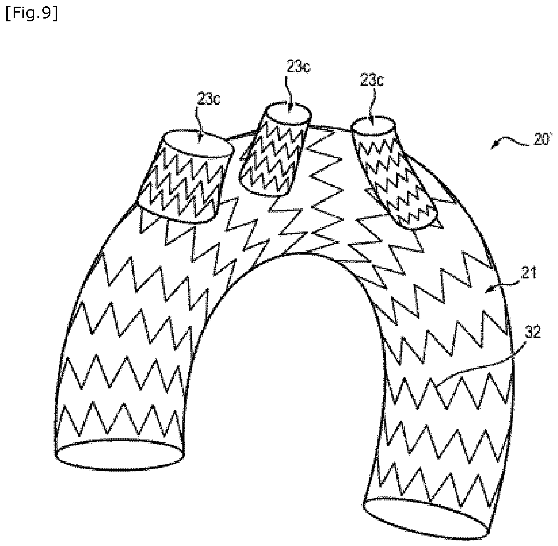

La **Figure 9** représente un exemple alternatif de structure d'empreinte 20' pour l'obtention d'une paroi de prothèse.

La structure d'empreinte 20' est un mandrin plein sur lequel une paroi de prothèse peut être fabriquée par moulage, avec une pluralité de branches de prothèse.

La structure d'empreinte 20' comprend un tube 21 coudé correspondant à la voie principale de la crosse aortique.

Elle comprend en outre trois empreintes 23c correspondant à trois branches de la crosse aortique.

Des fils métalliques d'un maillage métallique 32 sont disposés sur le tube 21' et sur les empreintes 23c.

Dans cet exemple, les empreintes 23c s'étendent sur une longueur plus importante relativement à une longueur du tube 21, comparativement aux protrusions 23a de la structure d'empreinte de la Figure 4. Notamment, l'empreinte 23c située à droite de la figure présente une extension longitudinale supérieure à 10% de l'extension longitudinale du tube 21 suivant la ligne centrale.

## Revendications

1. Procédé de fabrication d'une endoprothèse vasculaire destinée à être insérée dans une cavité naturelle d'un individu, à partir d'un modèle tridimensionnel (10) de la cavité naturelle, ledit modèle comprenant une voie principale (11), au moins une branche (12) de voie s'étendant depuis la voie principale (11) et une intersection (13) entre la voie principale (11) et la branche (12) de voie,
le procédé comprenant des étapes de :
- obtention (300) d'une structure (20) d'empreinte conformée pour suivre une forme de la voie principale (11),
la structure d'empreinte (20) comprenant au moins un emplacement en correspondance avec l'intersection (13) du modèle tridimensionnel,
- placement (400) d'un renforcement (33) sur ledit emplacement de la structure d'empreinte (20),
- fabrication (500) d'une paroi (31) de prothèse au moyen de la structure d'empreinte (20), la paroi de prothèse étant en polymère, une fenêtre de prothèse ou une branche de prothèse étant fabriquée au niveau dudit emplacement de la structure d'empreinte (20),
- obtention (600) de l'endoprothèse (30-1) vasculaire comprenant la fenêtre de prothèse ou la branche de prothèse.

2. Procédé de fabrication d'une endoprothèse selon la revendication 1, dans lequel la structure d'empreinte (20) est obtenue par impression tridimensionnelle, par exemple par dépôt de matière fondue ou FDM.

3. Procédé de fabrication d'une endoprothèse selon l'une quelconque des revendications 1 ou 2, dans lequel la voie principale (11) comporte une ligne centrale (L), et dans lequel une extension radiale de la structure d'empreinte (20) par rapport à une courbe longitudinale image de la ligne centrale est compris entre 101% et 130%, et de préférence entre 110% et 120%, d'une extension radiale de la voie principale (11) par rapport à la ligne centrale (L).

4. Procédé de fabrication d'une endoprothèse selon l'une quelconque des revendications 1 à 3, dans lequel la structure d'empreinte (20) est pleine et la paroi (31) de prothèse est fabriquée par moulage par-dessus la structure d'empreinte.

5. Procédé de fabrication d'une endoprothèse selon la revendication 4, dans lequel ledit emplacement de la structure d'empreinte (20) comprend un volume cylindrique formant une protrusion (23a), le placement (400) du renforcement (33) comprenant le positionnement du renforcement (33) contre la protrusion (23a).

6. Procédé de fabrication d'une endoprothèse selon l'une quelconque des revendications 1 à 5, dans lequel la fabrication (500) de la paroi (31) de prothèse comprend un enroulement de filaments de polymère autour de la structure d'empreinte (20), et/ou un enrobage de la structure d'empreinte (20) par des couches de polymère.

7. Procédé de fabrication d'une endoprothèse selon l'une quelconque des revendications 1 à 6, comprenant une étape intermédiaire de placement (450), le long de la structure d'empreinte (20), d'un maillage métallique (32) destiné à s'étendre le long d'au moins une partie de l'endoprothèse vasculaire, la paroi (31) de prothèse étant réalisée autour du maillage métallique (32).

8. Procédé de fabrication d'une endoprothèse selon l'une quelconque des revendications 1 à 7, le renforcement comprenant un ergot (33), l'ergot étant de préférence au moins en partie fabriqué en nitinol.

9. Procédé de fabrication d'une endoprothèse selon l'une quelconque des revendications 1 à 8, dans lequel la structure d'empreinte (20) est entraînée en rotation pendant la fabrication (500) de la paroi (31) de prothèse.

10. Procédé de fabrication d'une endoprothèse selon l'une quelconque des revendications 1 à 9, dans lequel des capteurs (42) sont placés contre la structure d'empreinte (20) avant la fabrication (500) de la paroi de prothèse.

11. Procédé de fabrication d'une endoprothèse selon l'une quelconque des revendications 1 à 10, dans lequel la paroi (31) de prothèse est composée en majorité de polytétrafluoro-éthylène dit PTFE ou de poly-téréphtalate d'éthylène dit PETE, et/ou dans lequel le renforcement (33) est composé en majorité de nitinol.

12. Procédé de fabrication d'une endoprothèse selon l'une quelconque des revendications 1 à 11, le procédé comprenant une étape supplémentaire de fabrication d'une paroi de branche au moyen d'une empreinte de branche, la paroi de branche étant en polymère,
l'empreinte de branche étant placée au niveau de l'emplacement lors de la fabrication de la paroi de branche,
l'endoprothèse vasculaire obtenue comprenant la paroi de branche reliée à la paroi de prothèse.

13. Procédé de fabrication d'une endoprothèse selon l'une quelconque des revendications 1 à 12, dans lequel le modèle tridimensionnel (10) de la cavité naturelle comprend une discontinuité de la voie principale (11), telle qu'une tortuosité, une calcification ou un thrombus.

14. Endoprothèse vasculaire, par exemple une endoprothèse d'aorte, comprenant une fenêtre (41) et/ou une branche, l'endoprothèse étant obtenue par un procédé selon l'une quelconque des revendications 1 à 13.

15. Ensemble de fabrication d'une endoprothèse vasculaire, l'ensemble étant configuré pour mettre en œuvre un procédé de fabrication selon l'une quelconque des revendications 1 à 13, l'ensemble comprenant :
- une unité (51) d'obtention d'une structure d'empreinte par impression tridimensionnelle ;
- une unité (52) de fabrication de paroi de prothèse à partir de la structure d'empreinte ;
- une unité (53) de traitement, configurée pour contrôler la fabrication de la structure d'empreinte par l'unité de fabrication en fonction d'un modèle tridimensionnel de cavité naturelle, l'unité de traitement comprenant une mémoire (54) pour sauvegarder le modèle tridimensionnel.

16. Ensemble de fabrication d'une endoprothèse selon la revendication 15, comprenant en outre une unité d'acquisition de modèle (55) configurée pour acquérir des images d'une cavité naturelle d'un patient et pour générer à partir desdites images un modèle tridimensionnel (10) de la cavité naturelle.

## Patentansprüche

1. Verfahren zur Herstellung einer Gefäß-Endoprothese, die dazu bestimmt ist, in einen natürlichen Hohlraum eines Individuums eingeführt zu werden, ausgehend von einem dreidimensionalen Modell (10) des natürlichen Hohlraums, wobei das Modell einen Hauptweg (11), mindestens einen sich vom Hauptweg (11) erstreckenden Zweig (12) und einen Schnittpunkt (13) zwischen dem Hauptweg (11) und dem Zweig (12) umfasst,
wobei das Verfahren die folgenden Schritte umfasst:
- Erhalten (300) einer Abdruckstruktur (20), die so geformt ist, dass sie einer Form des Hauptweges (11) folgt,
wobei die Abdruckstruktur (20) mindestens eine Stelle umfasst, die dem Schnittpunkt (13) des dreidimensionalen Modells entspricht,
- Anbringen (400) einer Verstärkung (33) an der genannten Stelle der Abdruckstruktur (20),
- Herstellen (500) einer Prothesenwand (31) mittels der Abdruckstruktur (20), wobei die Prothesenwand aus Polymer besteht und an der Stelle der Abdruckstruktur (20) ein Prothesenfenster oder ein Prothesenzweig hergestellt wird,
- Erhalten (600) der Gefäß-Endoprothese (30-1) mit dem Prothesenfenster oder dem Prothesenzweig.

2. Verfahren zur Herstellung einer Endoprothese nach Anspruch 1, wobei die Abdruckstruktur (20) durch dreidimensionales Drucken, beispielsweise durch Schmelzauftrag oder FDM, erhalten wird.

3. Verfahren zur Herstellung einer Endoprothese nach einem der Ansprüche 1 oder 2, wobei der Hauptweg (11) eine Mittellinie (L) aufweist, und wobei eine radiale Ausdehnung der Abdruckstruktur (20) in Bezug auf eine Längskurve, die ein Abbild der Mittellinie ist, zwischen 101 % und 130 % und vorzugsweise zwischen 110 % und 120 % einer radialen Ausdehnung des Hauptwegs (11) in Bezug auf die Mittellinie (L) beträgt.

4. Verfahren zur Herstellung einer Endoprothese nach einem der Ansprüche 1 bis 3, wobei die Abdruckstruktur (20) massiv ist und die Prothesenwand (31) durch Formen über der Abdruckstruktur hergestellt wird.

5. Verfahren zur Herstellung einer Endoprothese nach Anspruch 4, wobei die Stelle der Abdruckstruktur (20) ein zylindrisches Volumen umfasst, das einen Vorsprung (23a) bildet, wobei das Anbringen (400) der Verstärkung (33) das Positionieren der Verstärkung (33) gegen den Vorsprung (23a) umfasst.

6. Verfahren zur Herstellung einer Endoprothese nach einem der Ansprüche 1 bis 5, wobei die Herstellung (500) der Prothesenwand (31) das Wickeln von Polymerfilamenten um die Abdruckstruktur (20) und/oder das Umhüllen der Abdruckstruktur (20) mit Polymerschichten umfasst.

7. Verfahren zur Herstellung einer Endoprothese nach einem der Ansprüche 1 bis 6, umfassend einen Zwischenschritt des Anbringens (450) eines Metallgitters (32) entlang der Abdruckstruktur (20), das sich entlang mindestens eines Teils der Gefäß-Endoprothese erstrecken soll, wobei die Prothesenwand (31) um das Metallgitter (32) herum hergestellt wird.

8. Verfahren zur Herstellung einer Endoprothese nach einem der Ansprüche 1 bis 7, wobei die Verstärkung einen Zapfen (33) umfasst, wobei der Zapfen vorzugsweise zumindest teilweise aus Nitinol hergestellt ist.

9. Verfahren zur Herstellung einer Endoprothese nach einem der Ansprüche 1 bis 8, wobei die Abdruckstruktur (20) während der Herstellung (500) der Prothesenwand (31) in Drehung versetzt wird.

10. Verfahren zur Herstellung einer Endoprothese nach einem der Ansprüche 1 bis 9, wobei vor der Herstellung (500) der Prothesenwand Sensoren (42) an der Abdruckstruktur (20) angebracht werden.

11. Verfahren zur Herstellung einer Endoprothese nach einem der Ansprüche 1 bis 10, wobei die Prothesenwand (31) überwiegend aus Polytetrafluorethylen, auch PTFE genannt, oder Polyethylenterephthalat, auch PETE genannt, besteht und/oder wobei die Verstärkung (33) überwiegend aus Nitinol besteht.

12. Verfahren zur Herstellung einer Endoprothese nach einem der Ansprüche 1 bis 11, wobei das Verfahren einen zusätzlichen Schritt der Herstellung einer Zweigwand mittels eines Zweigabdrucks umfasst, wobei die Zweigwand aus Polymer besteht,
der Zweigabdruck bei der Herstellung der Zweigwand an der Stelle platziert wird, wobei die erhaltene Gefäß-Endoprothese die mit der Prothesenwand verbundene Zweigwand umfasst.

13. Verfahren zur Herstellung einer Endoprothese nach einem der Ansprüche 1 bis 12, wobei das dreidimensionale Modell (10) des natürlichen Hohlraums eine Unterbrechung des Hauptwegs (11) umfasst, wie beispielsweise eine Windung, eine Verkalkung oder einen Thrombus.

14. Gefäß-Endoprothese, beispielsweise eine Aorten-Endoprothese, umfassend ein Fenster (41) und/oder einen Zweig, wobei die Endoprothese durch ein Verfahren nach einem der Ansprüche 1 bis 13 erhalten ist.

15. Vorrichtung zur Herstellung einer Gefäß-Endoprothese, wobei die Vorrichtung zur Durchführung eines Herstellungsverfahrens nach einem der Ansprüche 1 bis 13 konfiguriert ist und Folgendes umfasst:
- eine Einheit (51) zur Erzeugung einer Abdruckstruktur durch dreidimensionales Drucken;
- eine Einheit (52) zur Herstellung einer Prothesenwand aus der Abdruckstruktur;
- eine Verarbeitungseinheit (53), die so konfiguriert ist, dass sie die Herstellung der Abdruckstruktur durch die Herstellungseinheit auf der Grundlage eines dreidimensionalen Modells eines natürlichen Hohlraums steuert, wobei die Verarbeitungseinheit einen Speicher (54) zum Speichern des dreidimensionalen Modells umfasst.

16. Endoprothesen-Herstellungsvorrichtung nach Anspruch 15, die ferner eine Modell-Erfassungseinheit (55) umfasst, die so konfiguriert ist, dass sie Bilder eines natürlichen Hohlraums eines Patienten erfasst und aus diesen Bildern ein dreidimensionales Modell (10) des natürlichen Hohlraums erzeugt.

## Claims

1. Method for manufacturing a vascular endoprosthesis designed to be inserted into a natural cavity of an individual, based on a three-dimensional model (10) of the natural cavity, said model comprising a main passage (11), at least one passage branch (12) extending from the main passage (11), and an intersection (13) between the main passage (11) and the passage branch (12),
the method comprising steps of:
- obtaining (300) a mould structure (20) shaped to follow a form of the main passage (11),
the mould structure (20) comprising at least one location corresponding to the intersection (13) of the three-dimensional model,
- placing (400) a reinforcement (33) at said location of the mould structure (20),
- manufacturing (500) a prosthetic wall (31) using the mould structure (20), with the prosthetic wall made of polymer, and a prosthetic window or a prosthetic branch being produced at said location of the mould structure (20),
- obtaining (600) the vascular endoprosthesis (30-1) comprising the prosthetic window or prosthetic branch.

2. Method for manufacturing an endoprosthesis according to Claim 1, wherein the mould structure (20) is obtained by three-dimensional printing, for example by fused deposition modelling or FDM.

3. Method for manufacturing an endoprosthesis according to either one of Claims 1 or 2, wherein the main passage (11) includes a centreline (L), and wherein a radial extension of the mould structure (20) relative to a longitudinal curve which is an image of the centreline is between 101% and 130%, and preferably between 110% and 120%, of a radial extension of the main passage (11) relative to the centreline (L).

4. Method for manufacturing an endoprosthesis according to any one of Claims 1 to 3, wherein the mould structure (20) is solid and the prosthetic wall (31) is manufactured by moulding over the mould structure.

5. Method for manufacturing an endoprosthesis according to Claim 4, wherein said location of the mould structure (20) comprises a cylindrical volume that forms a protrusion (23a), with the placement (400) of the reinforcement (33) comprising positioning the reinforcement (33) against the protrusion (23a).

6. Method for manufacturing an endoprosthesis according to any one of Claims 1 to 5, wherein the manufacturing (500) of the prosthetic wall (31) comprises winding polymer filaments around the mould structure (20), and/or coating the mould structure (20) with layers of polymer.

7. Method for manufacturing an endoprosthesis according to any one of Claims 1 to 6, comprising an intermediate step of placing (450) a metal mesh (32) along the mould structure (20), which is intended to extend along at least a portion of the vascular endoprosthesis, with the prosthetic wall (31) being formed around the metal mesh (32).

8. Method for manufacturing an endoprosthesis according to any one of Claims 1 to 7, the reinforcement comprising a projection (33), the projection preferably being at least partially made of nitinol.

9. Method for manufacturing an endoprosthesis according to any one of Claims 1 to 8, wherein the mould structure (20) is rotated during manufacturing (500) of the prosthetic wall (31).

10. Method for manufacturing an endoprosthesis according to any one of Claims 1 to 9, wherein sensors (42) are placed against the mould structure (20) prior to manufacturing (500) the prosthetic wall.

11. Method for manufacturing an endoprosthesis according to any one of Claims 1 to 10, wherein the prosthetic wall (31) is predominantly composed of polytetrafluoroethylene, referred to as PTFE, or polyethylene terephthalate, referred to as PETE, and/or wherein the reinforcement (33) is predominantly composed of nitinol.

12. Method for manufacturing an endoprosthesis according to any one of Claims 1 to 11, the method comprising an additional step of manufacturing a branch wall using a branch mould, the branch wall being made of polymer,
the branch mould being placed at the location during the manufacturing of the branch wall,
the obtained vascular endoprosthesis comprising the branch wall connected to the prosthetic wall.

13. Method for manufacturing an endoprosthesis according to any one of Claims 1 to 12, wherein the three-dimensional model (10) of the natural cavity comprises a discontinuity in the main passage(11), such as tortuosity, calcification, or a thrombus.

14. Vascular endoprosthesis, such as an aortic endoprosthesis, comprising a window (41) and/or a branch, the endoprosthesis being obtained by a method according to any of Claims 1 to 13.

15. Manufacturing assembly for a vascular endoprosthesis, the assembly being configured to carry out a manufacturing method according to any of Claims 1 to 13, the assembly comprising:
- a unit (51) for obtaining a mould structure by three-dimensional printing;
- a unit (52) for manufacturing a prosthetic wall from the mould structure;
- a processing unit (53), configured to control the production of the mould structure by the manufacturing unit based on a three-dimensional model of a natural cavity, the processing unit comprising a memory (54) for storing the three-dimensional model.

16. Manufacturing assembly for an endoprosthesis according to Claim 15, further comprising a model acquisition unit (55) configured to capture images of a natural cavity of a patient and to generate a three-dimensional model (10) of the natural cavity from said images.
